# FASCICULE DE BREVET EUROPEEN

(11) **EP 3 672 502 B1**
(45) Date de publication et mention de la délivrance du brevet: **18.12.2024**
(21) Numéro de dépôt: 18753216.3
(22) Date de dépôt: 20.08.2018
(51) Int. Cl.: A61B 17/15, A61B 17/17, A61B 17/80

(54) **DISPOSITIF CHIRURGICAL DE GUIDAGE, POUR ASSISTER UNE TECHNIQUE D'OSTEOTOMIE D'OUVERTURE**
CHIRURGISCHE FÜHRUNGSVORRICHTUNG ZUR UNTERSTÜTZUNG EINER OPEN-WEDGE-OSTEOTOMIE
SURGICAL GUIDE DEVICE FOR ASSISTING AN OPEN WEDGE OSTEOTOMY

(30) Priorité: 22.08.2017 FR 1757798
(43) Date de publication de la demande: 01.07.2020
(60) Demande divisionnaire: 20212300.6
(73) Titulaire: Newclip International, 2163 Luxembourg (LU)
(72) Inventeur: LARCHE, Grégoire, 49300 Cholet (FR); PODGORSKI, Jean-Pierre, 49230 St-Crespin Sur Moine (FR); BAUDRY, Frank, 44380 Pornichet (FR); BERTON, Philippe, 13090 Aix En Provence (FR)
(74) Mandataire: Jacobacci Coralis Harle
(86) Numéro de dépôt international: PCT/EP2018/072461
(87) Numéro de publication internationale: WO 2019/038240

(56) Documents cités:
- WO-A1-2017/070318
- AU-A1- 2014 262 239
- CN-U- 206 333 941
- US-A- 5 935 128
- US-A1- 2004 260 301
- US-A1- 2009 254 126
- US-A1- 2016 235 454

## Description

### DOMAINE TECHNIQUE AUQUEL SE RAPPORTE L'INVENTION

La présente invention concerne de manière générale le domaine de la chirurgie orthopédique.

Elle concerne en particulier les dispositifs chirurgicaux destinés à assister les techniques d'ostéotomie d'ouverture.

### ARRIERE-PLAN TECHNOLOGIQUE

L'ostéotomie désigne la section chirurgicale d'un os long, pour en modifier son axe, sa taille ou sa forme, à des fins thérapeutiques ou plastiques.

Parmi les techniques d'ostéotomie, il est notamment connu les techniques dites d'« ostéotomie d'ouverture » qui consistent à sectionner l'os à traiter par un trait de coupe, puis à écarter les berges du trait en y introduisant un coin osseux ou artificiel (métal, ciment, substitut osseux).

Une fois la correction obtenue, les deux fragments osseux sont solidement maintenus par un matériel d'ostéosynthèse, notamment une plaque d'ostéosynthèse tenue par des vis.

Une telle intervention chirurgicale est par exemple utile pour traiter certaines arthroses localisées au niveau du genou, afin de corriger une déformation du membre inférieur et d'obtenir un redressement du tibia. En particulier, l'ostéotomie tibiale de valgisation reste une intervention de choix pour le traitement d'une arthrose fémoro-tibiale interne sur genu varum.

La planification préopératoire est essentielle dans ce type d'opération, notamment pour déterminer précisément la valeur de la correction dans l'espace qui sera à réaliser via l'ouverture (angle HKA et pente tibiale notamment). Mais, à ce jour, cette planification reste un exercice difficile dans laquelle l'expérience de l'opérateur et l'empirisme compte encore beaucoup dans le succès de l'opération.

De même, au cours de l'opération, il est souvent difficile pour le chirurgien de suivre scrupuleusement le dossier de planification préopératoire, notamment quant à l'agencement du trait de coupe et de la plaque d'ostéosynthèse.

US2016235454 et CN206333941 décrivent des dispositifs de guidage selon le préambule de la revendication 1. US2009254126 décrit un système d'ostéotomie.

Il existe par conséquent un besoin de moyens permettant une assistance de l'opérateur tout au long de l'intervention d'ostéotomie d'ouverture, de manière à suivre efficacement la planification préopératoire.

### OBJET DE L'INVENTION

Afin de remédier à l'inconvénient précité de l'état de la technique, la présente invention propose un dispositif chirurgical de guidage tel que revendiqué ci-après. destiné à être rapporté contre un os d'un patient, pour assister une technique d'ostéotomie d'ouverture.

Les modes de réalisation préférés de l'invention sont décrits dans les revendications dépendantes. Des méthodes associées sont également décrites ici pour faciliter la compréhension de l'invention, mais elles ne font pas partie de l'invention revendiquée.

Un tel dispositif chirurgical de guidage a ainsi l'intérêt d'assister l'opérateur au cours de l'intervention d'ostéotomie d'ouverture, de manière à suivre précisément la planification préopératoire.

Tel que développé par la suite, l'agencement des orifices traversants du dispositif chirurgical de guidage prend en compte la technique d'ostéotomie d'ouverture planifiée et préalablement simulée via une acquisition scanner avec une reconstitution tridimensionnelle de la zone anatomique à corriger.

Cette approche permet ainsi d'anticiper :
- la position finale des emplacements de vis de la plaque d'ostéosynthèse rapportée pour figer les deux fragments osseux, et
- la position spatiale du trait de coupe osseux à réaliser pour générer l'ouverture souhaitée.

Le dispositif chirurgical de guidage consiste alors avantageusement en un instrument spécifique au patient (dit encore PSI pour « Patient Specific Instrument »). La conception de cet instrument utilise les technologies d'imagerie et des outils de planification préopératoire pour sa fabrication sur mesure, tenant compte de l'anatomie unique de chaque patient.

Ce dispositif chirurgical de guidage aide ainsi le chirurgien à effectuer des interventions de manière précise et sécuritaire.

D'autres caractéristiques non limitatives et avantageuses du dispositif chirurgical de guidage conforme à l'invention, prises individuellement ou selon toutes les combinaisons techniquement possibles, sont les suivantes :
- le dispositif chirurgical de guidage comporte deux bordures latérales dont l'une au moins comporte au moins une patte adaptée à être positionnée contre une surface dudit os, laquelle patte présente une capacité de déformation élastique ;
- la pièce proximale comporte au moins deux orifices traversants juxtaposés, indépendants, qui s'étendent dans une bande virtuelle destinée à être orientée transversalement par rapport à l'axe longitudinal de l'os ;
- la pièce proximale est dotée d'une protrusion supérieure, ayant préférentiellement la forme d'un téton cylindrique radio-opaque, destinée à aider le praticien à positionner correctement le guide à la bonne distance par rapport au plateau tibial du patient ;
- la pièce proximale comporte également un conduit tubulaire qui est adapté à guider l'insertion d'une broche au sein de l'os, lequel conduit tubulaire est ménagé directement sus-jacent de la fenêtre de guidage et il est orienté de sorte que son axe longitudinal soit parallèle au plan de la fenêtre de guidage pour positionner une broche participant au guidage du trait de coupe,
- la pièce distale comporte au moins deux orifices traversants juxtaposés, indépendant, s'étendant dans une bande virtuelle qui est au moins approximativement perpendiculaire à la bande virtuelle suivie par les orifices de la pièce proximale et qui est destinée à être orientée au moins approximativement parallèlement à l'axe longitudinal de l'os ;
- la pièce distale comporte au moins un orifice traversant en forme de conduit tubulaire qui est adapté à guider l'insertion d'une broche au sein de l'os : un conduit tubulaire proximal qui est ménagé directement sous-jacent de la fenêtre de guidage et qui est orienté de sorte que son axe longitudinal soit parallèle au plan de la fenêtre de guidage pour positionner une broche participant au guidage du trait de coupe, et/ou un conduit tubulaire distal qui est ménagé à distance de la fenêtre de guidage et qui est orienté de sorte que son axe longitudinal coupe le plan de la fenêtre de guidage pour positionner une broche de butée de coupe et/ou d'indication de charnière ; selon un mode de réalisation particulier, la pièce distale comporte au moins deux conduits tubulaires distaux qui sont ménagés à distance de la fenêtre de guidage et qui sont orientés de sorte que leurs axes longitudinaux respectifs coupent le plan de la fenêtre de guidage pour positionner chacun une broche de butée de coupe et/ou d'indication de charnière ;
- le dispositif chirurgical de guidage est réalisé dans un matériau plastique adapté à la fabrication additive et à une utilisation invasive au sein du corps humain.

L'invention propose également un matériel pour la mise en oeuvre d'une technique d'ostéotomie d'ouverture, selon la revendication 7.

L'invention concerne aussi un procédé pour l'obtention d'un dispositif chirurgical de guidage selon l'invention.

Ce procédé comprend :
a) une étape de simulation de la correction destinée à être appliquée à un os par une technique d'ostéotomie d'ouverture à partir des données suivantes :
   - données anatomiques relevées sur l'os à traiter,
   - données relatives à la plaque d'ostéosynthèse à poser, et
   - données de planification préopératoire, relatives à la correction destinée à être appliquée à cet os par la technique d'ostéotomie d'ouverture, et
b) une étape de fabrication du dispositif chirurgical de guidage, tenant compte des données de correction issues de ladite étape de simulation.

Selon une caractéristique avantageuse, l'étape de fabrication dudit dispositif chirurgical de guidage consiste en une étape de fabrication additive.

Il est aussi décrit un programme d'ordinateur comprenant des moyens de code de programme enregistrés sur un support lisible par un ordinateur, comprenant :
- des premiers moyens de code de programme, pour le chargement de données relatives à la correction destinée à être appliquée à un os par une technique d'ostéotomie d'ouverture, à partir de données anatomiques dudit os et de données relatives à la position finale des vis pour la fixation de la plaque d'ostéosynthèse dans l'os,
- des deuxièmes moyens de code de programme, pour générer des données de fabrication du dispositif chirurgical de guidage, lesquelles données de fabrication contiennent des données relatives à l'agencement des orifices traversants du dispositif chirurgical de guidage tenant compte desdites données de correction et desdites données de position finale des vis,
lorsque ledit programme d'ordinateur est exécuté par un ordinateur.

Un procédé chirurgical d'ostéotomie d'ouverture non revendiqué est décrit, lequel procédé chirurgical comprend :
a) une étape de préparation, au cours de laquelle le dispositif chirurgical de guidage est fixé contre le surface de l'os, et des trous sont générés dans ledit os en regard de chacun des orifices traversants dudit dispositif chirurgical de guidage (avec éventuellement le positionnement d'au moins une broche inférieure agencée pour intersecter la charnière du foyer d'ostéotomie) ;
b) une étape d'ostéotomie de l'os comprenant :
   - une première phase de coupe au sein de la fenêtre de guidage du dispositif chirurgical de guidage,
   - un retrait de la pièce proximale du dispositif chirurgical de guidage, et
   - une seconde phase de coupe, en appui sur la pièce distale du dispositif chirurgical de guidage,
c) une étape de retrait de la pièce distale du dispositif chirurgical de guidage ;
d) une étape de fixation de la plaque d'ostéosynthèse contre un fragment distal de l'os, sous-jacent du trait de coupe, laquelle plaque d'ostéosynthèse est agencée de sorte que ses orifices traversants viennent en regard de trous issus de l'étape a) qui sont ménagés dans ledit fragment distal ;
e) une étape d'ouverture progressive du foyer d'ostéotomie, jusqu'à obtention de la correction souhaitée correspondant à une mise en regard de trous issus de l'étape a) qui sont ménagés dans un fragment proximal de l'os et sus-jacent du trait de coupe, avec des orifices traversants de ladite plaque d'ostéosynthèse ; et
f) une étape de fixation de la plaque d'ostéosynthèse contre le fragment proximal de l'os.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue générale et en perspective d'un dispositif chirurgical de guidage selon l'invention, rapporté contre un os d'un patient destiné à subir un technique d'ostéotomie d'ouverture ;
- la figure 2 est une vue de face du dispositif chirurgical de guidage illustré sur la figure 1 ;
- la figure 3 est une vue en perspective du dispositif chirurgical de guidage illustré sur les figures 1 et 2 ;
- la figure 4 est une vue de dessous du dispositif chirurgical de guidage des figures 1 à 3 ;
- la figure 5 est une vue de face d'une pièce proximale constitutive du dispositif chirurgical de guidage des figures 1 à 4, destinée à coopérer avec un premier fragment osseux ;
- la figure 6 est une vue en perspective d'une pièce distale constitutive du dispositif chirurgical de guidage des figures 1 à 4, destinée à coopérer avec un second fragment osseux ;
- les figures 7 à 13 représentent les principales étapes lors de la mise en oeuvre du dispositif chirurgical de guidage, dans le cadre d'une intervention chirurgicale d'ostéotomie d'ouverture ;
- la figure 14 est une vue générale et en perspective d'un second dispositif chirurgical de guidage selon l'invention, rapporté contre un os d'un patient destiné à subir un technique d'ostéotomie d'ouverture, qui se distingue de celui illustré sur les figures 1 à 13 par la présence d'un troisième conduit tubulaire adapté au guidage d'une seconde broche inférieure ;
- la figure 15 est une vue générale et de dessus du second dispositif chirurgical de guidage selon l'invention, avec un plan de coupe transversal qui passe dans l'os et au niveau de la future charnière ;
- la figure 16 illustre une étape du procédé chirurgical mettant en oeuvre le second dispositif chirurgical de guidage, à savoir le guidage d'une vis canulée par la seconde broche inférieure pour son insertion dans la charnière.

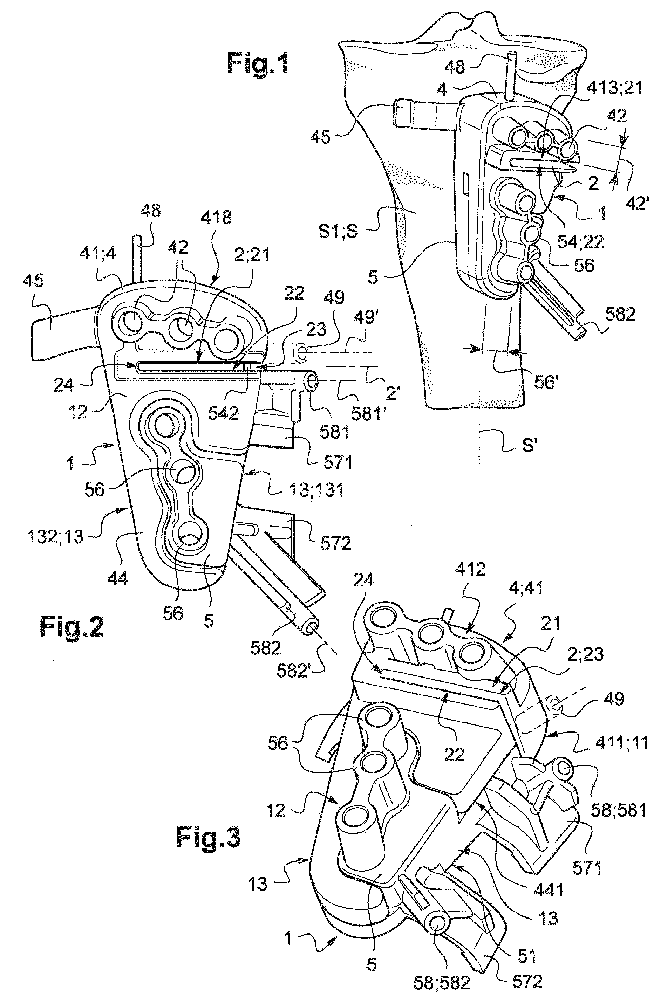

Tel que représenté sur la figure 1 notamment, le dispositif chirurgical de guidage 1 est destiné à être rapporté contre un os S d'un patient pour assister le chirurgien dans une technique d'ostéotomie d'ouverture.

Par « ostéotomie d'ouverture », on entend une intervention chirurgicale consistant à sectionner un os du patient par un trait de coupe, puis à écarter les berges du trait en y introduisant un coin osseux ou artificiel (métal, ciment, substitut osseux).

Cette technique chirurgicale peut être utilisée pour traiter différents os du corps humain.

Une telle intervention chirurgicale est par exemple utile pour traiter certaines arthroses localisées au niveau du genou, afin de corriger une déformation du membre inférieur et d'obtenir un redressement du tibia. En particulier, l'ostéotomie tibiale de valgisation reste une intervention de choix pour le traitement d'une arthrose fémoro-tibiale interne sur genu varum.

De manière générale, l'os S à traiter comporte une surface S1 ; et cet os S comporte également un axe longitudinal S'.

### Dispositif chirurgical de guidage

Le dispositif chirurgical de guidage 1, représenté plus en détails sur les figures 1 à 4, se présente sous la forme générale d'un bloc.

Ce dispositif chirurgical de guidage 1 (désigné encore sous le nom de guide) comprend un ensemble de faces :
- une face arrière 11, adaptée à être positionnée contre une surface S1 de l'os S à traiter,
- une face avant 12, située à l'opposé de ladite face arrière 11, et
- deux faces latérales 13, à savoir : une première face latérale 131 (postérieure) et une seconde face latérale 132 (antérieure).

Ce dispositif chirurgical de guidage 1 comporte encore une fenêtre 2, en forme de fente, pour le guidage du trait de coupe destiné à être réalisé dans l'os S.

Cette fenêtre de guidage 2 est délimitée par un bord supérieur 21 et par un bord inférieur 22, ménagés en regard et à distance l'un de l'autre.

Ces deux bords 21 et 22 sont rectilignes. Ils s'étendent parallèlement l'un par rapport à l'autre, définissant ensemble un plan général 2' (figure 2).

La distance entre les deux bords 21, 22 correspond avantageusement à l'épaisseur de la lame de coupe (au jeu fonctionnel près).

L'épaisseur des bords 21, 22 (entre les faces arrière 11 et avant 12) est avantageusement comprise entre 0,8 mm et 1,6 mm.

La fenêtre de guidage 2 est traversante, c'est-à-dire qu'elle débouche au niveau des faces arrière 11 et avant 12 du dispositif chirurgical de guidage 2.

Cette fenêtre de guidage 2 est en plus débouchante latéralement d'un côté seulement, au niveau d'une ouverture latérale 23 située dans la première face latérale 131 du dispositif chirurgical de guidage 1.

La fenêtre de guidage 2 comporte encore une bordure latérale 24, formant un fond, qui s'étend entre les deux bords 21, 22 et qui est située à l'opposé de l'ouverture latérale 23.

Le dispositif chirurgical de guidage 1 comprend ici deux pièces 4, 5 qui sont assemblées l'une avec l'autre par le biais de moyens de solidarisation amovible.

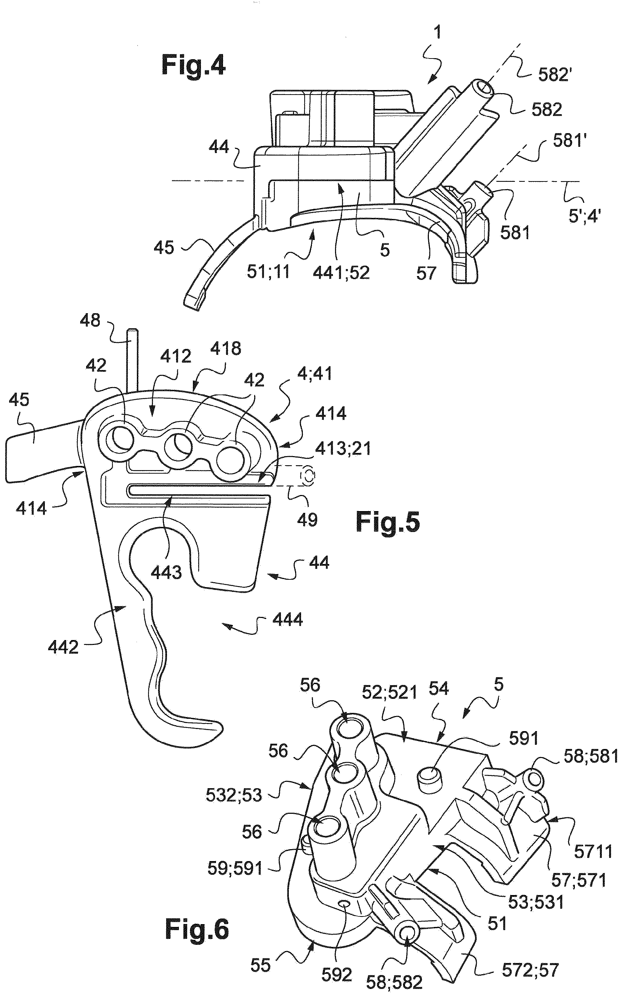

Plus précisément, ce dispositif chirurgical de guidage 1 comprend une première pièce 4, dite encore pièce ou partie proximale (figure 5), et une seconde pièce 5, dite encore pièce ou partie distale (figure 6).

Ces deux pièces 4, 5 sont destinées à coopérer chacune avec l'un des deux fragments osseux destinés à être générés par le trait de coupe réalisé dans l'os S au travers de la fenêtre de guidage 2.

Telles que décrites plus en détails ci-après, ces deux pièces 4, 5 sont chacune munies de plusieurs orifices traversants qui sont adaptés à accueillir des organes implantables temporairement dans l'os S et/ou à guider un perçage de trous dans cet osS.

Chacune de ces pièces 4, 5 est avantageusement réalisée monobloc dans un matériau plastique adapté aux techniques de fabrication additive (dite encore « impression 3D » ou « impression en trois dimensions »).

Ce matériau plastique est par exemple choisi parmi les polyamides.

### Première pièce

La première pièce 4 (pièce proximale), représentée plus en détails et isolément sur la figure 5, comporte une portion supérieure 41 qui présente :
- une face arrière 411, destinée à former une portion supérieure de la face arrière 11 du dispositif chirurgical de guidage 1 (figure 3), et
- une face avant 412, destinée à former une portion supérieure de la face avant 12 du dispositif chirurgical de guidage 1.

La portion supérieure 41 comporte plusieurs orifices traversants 42, débouchant au travers de ses faces arrière 411 et avant 412.

Les orifices traversants 42, dits encore « orifices traversants proximaux », sont adaptés à accueillir des organes implantables temporairement dans l'os S et/ou à guider un perçage de trous dans cet os S.

En l'espèce, ces orifices traversants 42 sont destinés notamment à servir de guide de perçage de trous dans un fragment proximal de l'os en vue de faciliter le montage ultérieur de la plaque d'ostéosynthèse.

Les orifices traversants 42, juxtaposés, sont au moins au nombre de deux (ici au nombre de trois).

Ces orifices traversants 42 s'étendent dans une bande virtuelle 42' destinée à être orientée transversalement par rapport à l'axe longitudinal S' de l'os S (figure 1).

Cette portion supérieure 41 comporte une bordure inférieure 413, destinée à former le bord supérieur 21 de la fenêtre de guidage 2.

Cette portion supérieure 41 est délimitée également par deux bordures latérales 414 formant une portion supérieure des faces latérales 13 du dispositif chirurgical de guidage 1.

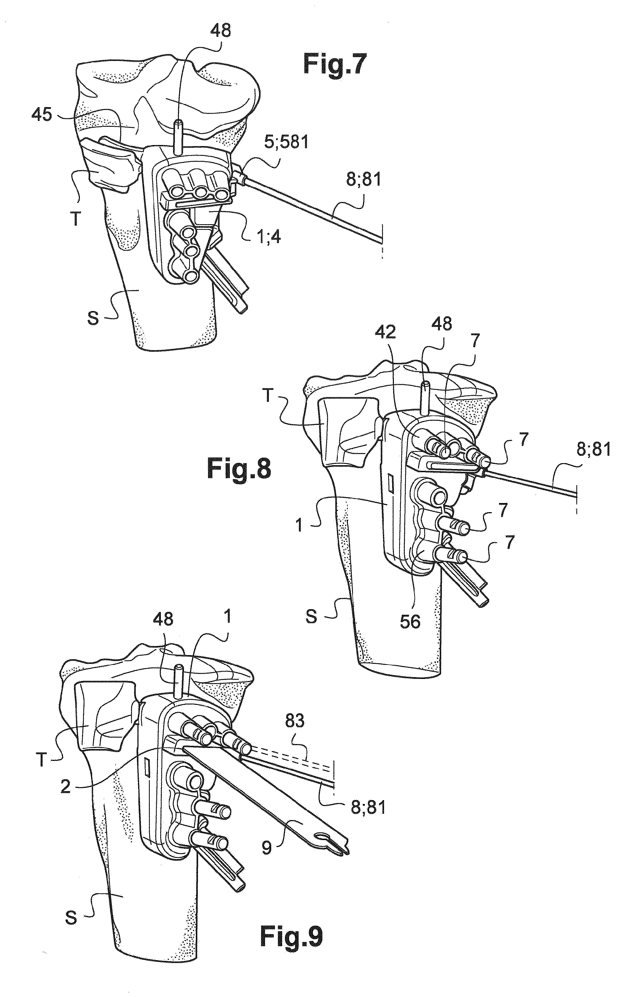

L'une de ces bordures latérales 414 comporte ici une patte latérale 45 (patte antérieure) qui est adaptée à être positionnée contre une surface de l'os S, tel que développé ci-après en relation avec les figures 7 à 13.

La patte 45 présente une capacité de déformation élastique, de sorte à épouser de manière optimale la surface de l'os S.

Cette patte 45 s'étend approximativement parallèlement à la bordure inférieure 413 et du côté de la face arrière 411 de la portion supérieure 41 (figure 4). Sa surface arrière correspond approximativement à la surface anatomique de l'os qu'elle est destinée à recouvrir.

La portion supérieure 41 est prolongée ici par une portion inférieure 44 qui est destinée à coopérer avec la seconde pièce 5 en vue de leur assemblage amovible.

La portion inférieure 44 se présente sous la forme générale d'une platine destinée à épouser une réservation complémentaire ménagée dans la face avant de la seconde pièce 5.

Cette portion inférieure 44 comporte alors :
- une face arrière 441, destinée à recouvrir la seconde pièce 5 du dispositif chirurgical de guidage 1 (figure 3), et
- une face avant 442, destinée à former une partie de la face avant 12 du dispositif chirurgical de guidage 1.

La face arrière 441 de la portion inférieure 44 est en retrait par rapport à la face arrière 411 de la portion supérieure 41.

Cette portion inférieure 44 est ici délimitée encore par une bordure supérieure 443 qui est destinée à former une partie du bord inférieur 22 et la bordure latérale 24 de la fenêtre de guidage 2, en particulier destinée à former la partie avant de ce bord inférieur 22.

Cette portion inférieure 44 comporte encore une lumière 444, au sein de laquelle est destinée à venir se placer une forme complémentaire de la seconde pièce 5, notamment une partie comportant les orifices traversants de cette seconde pièce 5.

La pièce proximale 4 est encore dotée d'une protrusion supérieure 48 qui est destinée à aider le praticien à positionner correctement le dispositif chirurgical de guidage 1 à la bonne distance par rapport au plateau tibial du patient (vérification de l'altitude du guide 1).

Cette protrusion supérieur 48 est rapportée, ici de manière amovible, au niveau d'une face supérieure 418 (libre) de la portion supérieure 41.

Cette protrusion supérieur 48 a préférentiellement la forme d'un téton cylindrique (dite encore tige ou broche) radio-opaque.

Par ailleurs, de manière optionnelle, cette première pièce 4 comporte également un conduit tubulaire 49 adapté à la réception d'une broche additionnelle destinée à être implantée temporairement dans l'os S pour assister la technique d'ostéosynthèse (ce conduit tubulaire 49 est représenté en traits discontinus sur les figures 2, 3 et 5).

Ce conduit tubulaire 49 est ménagé ici dans la portion supérieure 41, au travers de son autre bordure latérale 414 et à proximité de sa bordure inférieure 413 (destinée à former le bord supérieur 21 de la fenêtre de guidage 2).

Ce conduit tubulaire 49 comporte un axe longitudinal 49' qui est orienté parallèlement, ou au moins approximativement parallèlement, à la bordure inférieure 413 précitée (figure 2).

Ce conduit tubulaire 49 est ainsi ménagé directement sus-jacent de la fenêtre de guidage 2 et il est orienté de sorte que son axe longitudinal 49' soit parallèle au plan 2' de la fenêtre de guidage 2 pour positionner une broche participant au guidage du trait de coupe (cette broche 83 « additionnelle » est représentée schématiquement sur la figure 9).

Ce conduit tubulaire 49 s'étend parallèlement au plan défini par le bord supérieur 21 de la fenêtre de guidage 2, avec un décalage (ou déport ou offset) compris entre 0,5 et 3 mm par rapport à ce bord supérieur 21 (de préférence de l'ordre de 1 mm).

Ce conduit tubulaire 49 définit encore un angle compris entre 10° et 30° par rapport au plan général de la première pièce 4' (figure 4).

### Seconde pièce

La seconde pièce 5, illustrée isolément sur la figure 6, comporte également plusieurs faces :
- deux faces opposées : une face arrière 51 et une face avant 52,
- deux faces latérales 53 : une première face latérale 531 (à droite sur la figure 6) et une seconde face latérale 532 (à gauche sur le figure 6), formant une portion inférieure des faces latérales 13 du dispositif chirurgical de guidage 1, et
- deux faces d'extrémité : une face proximale 54 et une face distale 55.

La face arrière 51 est destinée à former une portion inférieure de la face arrière 11 du dispositif chirurgical de guidage 1 (figure 3).

La face avant 52 comporte une réservation 521 destinée à recevoir la portion inférieure 44 de la première pièce 4.

La face proximale 54 constitue une bordure supérieure de la seconde pièce 5, qui est destinée à former une partie du bord inférieur 22 de la fenêtre de guidage 2 (en l'occurrence une partie arrière de ce bord inférieur 22) en combinaison ici avec la bordure supérieure 443 de la portion inférieure 44 de la première pièce 4.

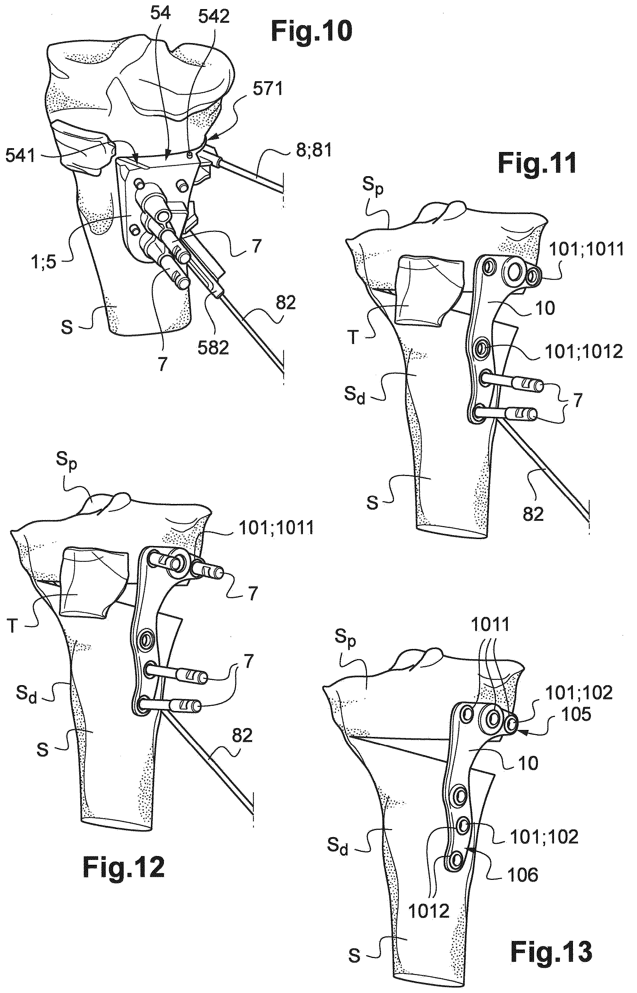

Un emplacement 541 dédié au guidage d'une coupe bi-planaire peut être prévue au niveau de cette face proximale 54 (visible figure 10).

Cet emplacement 541 est ajouté au dispositif chirurgical de guidage 1 lorsque le trait de coupe se retrouve en conflit avec la tubérosité tibiale. Il est alors nécessaire de préserver l'insertion du tendon rotulien par ce procédé.

Cet emplacement 541 se situe ici du côté opposé à l'ouverture latérale 23.

Cette face proximale 54 comporte encore, du côté de l'ouverture latérale 23, des moyens entretoises 542 pour maintenir l'écartement entre les deux bords 21, 22 de la fenêtre de guidage 2 (figures 2 et 10).

Ces moyens entretoises 542 consistent par exemple en un pion monobloc équipant la face proximale 54, et venant en appui contre la bordure inférieure 413 de la première pièce 4.

La seconde pièce 5 comporte plusieurs orifices traversants 56 qui sont adaptés à accueillir des organes implantables temporairement dans l'os S et/ou à guider un perçage de trous dans cet os S.

En l'espèce, cette seconde pièce 5 comporte des orifices traversants 56, dits encore « orifices traversants distaux » qui sont destinés notamment à servir de guide de perçage de trous dans un fragment distal de l'os en vue de faciliter le montage ultérieur de la plaque d'ostéosynthèse.

Ces orifices traversants 56 sont débouchants au travers des faces arrière 51 et avant 52 de la seconde pièce 5.

Ces orifices traversants 56, juxtaposés, sont au moins au nombre de deux (ici au nombre de trois).

Ces orifices traversants 56 s'étendent dans une bande virtuelle 56' destinée à être orientée approximativement parallèlement par rapport à l'axe longitudinal S' de l'os (figure 1).

La première face latérale 531 de cette seconde pièce 5 comporte des pattes 57 destinée à être positionnées contre la surface de l'os S, tel que développé ci-après en relation avec les figures 7 à 13.

Ces pattes 57 ont une face arrière qui correspond approximativement à la surface anatomique de l'os qu'elles sont destinées à recouvrir ; elles présentent une capacité de déformation élastique, de sorte à épouser de manière optimale la surface de l'os S.

Ces pattes 57 s'étendent approximativement parallèlement à la face proximale 54 et du côté de la face arrière 51.

En l'espèce, cette seconde pièce 5 comporte deux pattes :
- une patte supérieure 571, ménagée le long de la face proximale 54, et
- une patte inférieure 572, ménagée du côté de la face distale 55.

En particulier, la patte supérieure 571 comporte une bordure supérieure 5711 s'étendant dans le prolongement de la face proximale 54 de manière à participer à former la surface de guidage pour le trait de coupe.

Par ailleurs, cette seconde pièce 5 comporte également des conduits tubulaires 58 adaptés à la réception de broches destinées à être implantées temporairement dans l'os S pour assister la technique d'ostéosynthèse.

Un premier conduit tubulaire 581, supérieur ou proximal (dit encore « fût supérieur »), est ménagé ici au travers de la patte supérieure 571.

Ce conduit tubulaire supérieur 581 comporte un axe longitudinal 581' qui est orienté parallèlement, ou au moins approximativement parallèlement, à la face proximale 54 (figure 2).

Cet axe longitudinal 581' est en plus avantageusement orienté parallèlement, ou au moins approximativement parallèlement, par rapport à l'axe longitudinal 49' du conduit tubulaire 49 de la première pièce 4.

Ce conduit tubulaire proximal 581 est ménagé directement sous-jacent de la fenêtre de guidage 2 et il est orienté de sorte que son axe longitudinal 581' soit parallèle au plan 2' de la fenêtre de guidage 2.

Ce conduit tubulaire supérieur 581 est adapté à guider une broche supérieure qui est destinée à participer au guidage du trait de coupe.

Ce conduit tubulaire supérieur 581 s'étend parallèlement au plan défini par le bord inférieur 22 de la fenêtre de guidage 2, avec un décalage (ou déport ou offset) compris entre 0,5 et 3 mm par rapport à ce bord inférieur 22 (de préférence de l'ordre de 1 mm).

Ce conduit tubulaire supérieure 581 définit encore un angle compris entre 10° et 30° par rapport au plan général de la seconde pièce 5' (figure 4).

Un second conduit tubulaire 582, inférieur ou distal (dit encore « fût inférieur » ou « premier conduit tubulaire distal » ou « premier conduit tubulaire inférieur »), est ménagé au travers de la patte inférieure 572.

Ce conduit tubulaire inférieur 582 est orienté de sorte que son axe longitudinal 582' coupe le plan général 2' de la fenêtre de guidage 2 (figure 2).

Ce point d'intersection est destiné à se situer dans l'encombrement de l'os, en particulier au niveau du foyer d'ouverture.

Le point d'intersection permet de localiser un point théorique situé à une distance déterminée de la surface de l'os et du plateau (par exemple, le point théorique est situé de 10 à 11 mm de la corticale externe).

Ce conduit tubulaire inférieur 582 est destiné à guider une broche inférieure, ascendante, servant de butée de coupe et/ou d'indication de charnière.

L'orientation de l'axe longitudinal 582' de ce conduit tubulaire inférieur 582 peut être définie par deux angles :
- un premier angle d'inclinaison défini entre l'axe longitudinal 582' et le plan général 2' de la fenêtre de guidage 2, compris entre 40° et 70° (figure 2), et
- un second angle d'inclinaison défini entre l'axe longitudinal 582' et le plan général de cette seconde pièce 5', compris entre 35° et 70° (figure 4).

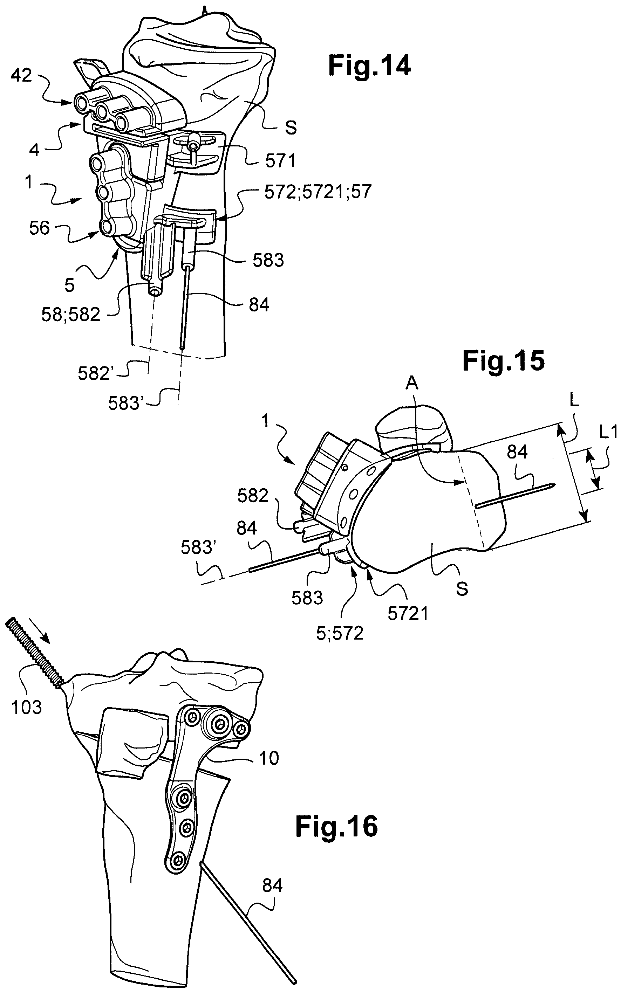

Les figures 14 et 15 représentent un second dispositif chirurgical de guidage selon l'invention, variante du dispositif chirurgical décrit ci-dessus en relation avec les figures 1 à 6.

Le second dispositif chirurgical de guidage 1 se distingue uniquement par la présence d'un troisième conduit tubulaire 583 (dit encore « second conduit tubulaire inférieur » ou « second conduit tubulaire distal »), ménagé sur la seconde pièce 5 pour la réception d'une broche destinée à être implantée temporairement dans l'os S pour assister la technique d'ostéosynthèse.

Le troisième conduit tubulaire 583, également inférieur ou distal (dit encore « second fût inférieur »), est ménagé ici au travers de la patte inférieure 572.

Ce troisième conduit tubulaire 583 est ici agencé entre le second conduit tubulaire 582 et l'extrémité libre 5721 de cette patte inférieure 572. De manière alternative non représentée, en fonction de l'emplacement primaire du second conduit tubulaire 582, le troisième conduit tubulaire 583 peut également être disposé entre la première face latérale 531 de la seconde pièce 5 et le second conduit tubulaire 582 ; c'est par exemple le cas lorsque ce second conduit tubulaire 582 est déporté et décentré pour tenir compte du changement de pente tibiale demandé (en effet, plus on ouvre en postérieur, plus la première broche ira en antérieur et donc proche de l'extrémité libre 5721 de la patte inférieure 572).

Ce troisième conduit tubulaire 583 est orienté de sorte que son axe longitudinal 583' coupe le plan général 2' de la fenêtre de guidage 2 (figure 2).

Son point d'intersection est destiné à se situer dans l'encombrement de l'os, en particulier au niveau du foyer d'ouverture et plus précisément encore au niveau de la charnière.

Le point d'intersection permet de localiser un point théorique situé à une distance déterminée de la surface de l'os et du plateau (par exemple, le point théorique est situé de 10 à 11 mm de la corticale externe).

Ce troisième conduit tubulaire 583 est destiné à guider une seconde broche inférieure, ascendante. Tel qu'illustré sur la figure 16, la seconde broche inférieure est également utile pour guider l'insertion d'une vis canulée au travers de la charnière.

L'orientation de l'axe longitudinal 583' de ce troisième conduit tubulaire 583 est avantageusement parallèle à l'axe longitudinal 582' du second conduit tubulaire 582.

### Moyens de solidarisation amovible

Les deux pièces 4, 5 sont solidarisées ensemble par le biais de moyens de solidarisation amovibles 59, en l'espèce sous la forme de structures d'emboîtement complémentaires.

A cet effet, au niveau de sa réservation 521, la face avant 51 de la seconde pièce 5 comporte des tenons 591 (figure 6) destinés à coopérer par emboîtement avec des mortaises complémentaires (non visibles) qui sont ménagées au niveau de la face arrière 441 de la portion distale 44 de la première pièce 4.

La seconde pièce 5 est encore munie d'ergots 592 par exemple en forme de calottes sphériques ou pions coniques (figure 6) qui sont judicieusement répartis de sorte à coopérer par emboîtement élastique avec des logements complémentaires (non visibles) équipant la première pièce 4.

Ici, ces ergots et logements complémentaires sont ménagés sur les chants en regard, d'une part, de la lumière 444 de la première pièce 4 et, d'autre part, de la réservation 521 de la seconde pièce 5.

### Matériel pour technique d'ostéotomie d'ouverture

Tel que représenté sur les figures 7 et suivantes, le dispositif chirurgical de guidage 1 est destiné à être utilisé en combinaison avec un ensemble d'autres éléments pour la mise en oeuvre de la technique d'ostéotomie d'ouverture.

Le matériel correspondant comprend ainsi avantageusement :
- des organes implantables temporairement dans l'os, au travers des orifices traversants 42, 56 de ce dispositif chirurgical de guidage 1, à savoir notamment des axes rigides 7 (appelés « pins »),
- des broches 8 implantables temporairement dans l'os S, au travers des conduits traversants 49, 58 dudit dispositif chirurgical de guidage 1,
- une lame de coupe 9 (figure 9), associée à un moteur chirurgical, pour réaliser le trait de coupe guidé par le dispositif chirurgical de guidage 1,
- une plaque d'ostéosynthèse 10 (figure 11 à 13), munie d'orifices traversants 101 pour l'accueil de vis de fixation 102 destinés à être implantées dans l'os S, et
- un jeu de vis 102 pour la fixation dans l'os S, destiné à être inséré dans les orifices traversants 101 de la plaque d'ostéosynthèse 10 pour fixer cette dernière à la surface de l'os S.

La plaque d'ostéosynthèse 10 est destinée à être solidarisée avec les deux fragments osseux, suite à la technique d'ostéotomie d'ouverture guidée par le dispositif chirurgical de guidage 1, de manière à figer la correction apportée par le chirurgien.

Une telle plaque d'ostéosynthèse 10 est par exemple décrite en détails dans le document FR2980967.

Tel que décrit ci-dessous en relation avec la figure 13, cette plaque d'ostéosynthèse 10 se compose de deux parties allongées, présentant ici ensemble une forme générale de L, à savoir :
- une partie proximale 105, destinée à être positionnée contre un fragment proximal Sp de l'os S, et
- une partie distale 106, destinée à être positionnée contre un fragment distal Sd de ce même os S.

Chacune de ces deux parties 105, 106 est munie d'un groupe d'orifices traversants 1011, 1012 pour l'accueil de vis de fixation 102.

Selon l'invention, l'agencement de chaque groupe d'orifices traversants 42, 56 du dispositif chirurgical de guidage 1 correspond à l'agencement de l'un des deux groupes d'orifices traversants 1011, 1012 de la plaque d'ostéosynthèse 1 :
- l'agencement des orifices traversants 42 de la première pièce 4 correspond à l'agencement des orifices traversants 1011 de la partie proximale 105 de la plaque d'ostéosynthèse 10, et
- l'agencement des orifices traversants 56 de la seconde pièce 5 correspond à l'agencement des orifices traversants 1012 de la partie distale 106 de la plaque d'ostéosynthèse 10.

Encore selon l'invention, l'agencement relatif entre les deux groupes d'orifices traversants 42, 56 du dispositif chirurgical de guidage 1 est fonction, d'une part, de la correction destinée à être appliquée lors de la technique d'ostéotomie d'ouverture et, d'autre part, de l'agencement relatif des deux groupes d'orifices traversants 1011, 1012 de la plaque d'ostéosynthèse 1.

### Fabrication du dispositif chirurgical de guidage

La fabrication du dispositif chirurgical de guidage 1 est avantageusement réalisée sur mesure pour le patient. Ce dispositif chirurgical de guidage 1 consiste ainsi avantageusement en un instrument spécifique au patient ou « PSI ».

La fabrication de ce dispositif chirurgical de guidage 1, et en corollaire de ses deux pièces 4, 5 constitutives, tient compte avantageusement des données suivantes :
- de données anatomiques relevées sur l'os à traiter, avantageusement obtenues par une technique d'imagerie médicale (par exemple par scanner),
- de données relatives à la plaque d'ostéosynthèse 10 à poser, notamment de la position finale des emplacements des vis 102 pour la fixation de la plaque d'ostéosynthèse 10, et
- de données de planification préopératoire, relatives à la correction destinée à être appliquée à cet os par la technique d'ostéotomie d'ouverture.

Le procédé pour l'obtention du dispositif chirurgical de guidage 1 selon l'invention comprend avantageusement les étapes suivantes :
a/ une étape de simulation de la correction destinée à être appliquée à l'os S par une technique d'ostéotomie d'ouverture à partir des données précitées, puis
b/ une étape de fabrication proprement dite du dispositif chirurgical de guidage 1, tenant compte des données de correction issues de l'étape de simulation.

Pour la mise en oeuvre de l'étape de simulation, un outil de planification préopératoire est proposé sous la forme d'un programme d'ordinateur destiné à générer des données pour la fabrication du dispositif chirurgical de guidage 1 sur la base des données précitées.

En l'espèce, ce programme d'ordinateur comprend des moyens de code de programme enregistrés sur un support lisible par un ordinateur, comprenant :
- des premiers moyens de code de programme, pour le chargement de données relatives à la correction destinée à être appliquée à un os par une technique d'ostéotomie d'ouverture, à partir de données anatomiques de l'os à traiter et de données relatives à la position finale des vis pour la fixation dans l'os de la plaque d'ostéosynthèse, et
- des deuxièmes moyens de code de programme, pour générer des données de fabrication du dispositif chirurgical de guidage 1, lesquels données de fabrication contiennent des données relatives, d'une part, à la forme de la face arrière 11 du dispositif chirurgical de guidage 1 et, d'autre part, à l'agencement des orifices traversants 42, 56 du dispositif chirurgical de guidage 1 tenant compte desdites données de correction et desdites données de position finale des vis,
lorsque ledit programme d'ordinateur est exécuté par un ordinateur.

Les données anatomiques de l'os à traiter consistent par exemple en des données issues d'une acquisition scanner, à partir desquelles une modélisation tridimensionnelle de l'ensemble des parties impliquées dans le processus de correction est effectuée.

Les données de correction sont fournies par le chirurgien, dans le cadre de sa planification pré-opératoire.

La position finale des vis et de la plaque respecte quant à elle les principes usuels nécessaires à l'obtention de la performance mécanique recherchée pour ce matériel (notamment positionnement anatomique de l'implant).

Les deuxièmes moyens de code de programme consistent avantageusement en des moyens de modélisation tridimensionnelle qui sont destinés à générer les données de fabrication du dispositif chirurgical de guidage 1 sous forme d'un « fichier tridimensionnel » (ou « fichier 3D ») qui correspond à un modèle tridimensionnel exploitable par une imprimante tridimensionnelle.

Tel qu'illustré en relation avec les figures 7 et suivantes, dans ces données de fabrication, l'agencement des orifices traversants 42, 56 du dispositif chirurgical de guidage 1 prend en compte la technique d'ostéotomie d'ouverture planifiée et anticipe la position finale des emplacements de vis de la plaque d'ostéosynthèse 10 rapportée pour figer les deux fragments osseux Sp et Sd.

L'étape de fabrication du dispositif chirurgical de guidage 1 consiste en une étape de fabrication additive.

### Procédé chirurgical d'ostéotomie d'ouverture non revendiqué

Une première étape du procédé chirurgical consiste en une étape de préparation (figures 7 et 8).

Au cours de cette étape de préparation, le dispositif chirurgical de guidage 1 (avec ses deux parties 4 et 5 assemblées) est positionné de sorte que sa face arrière 11 vient prendre appui de la surface de l'os S, en lieu et place de l'emplacement futur de la plaque d'ostéosynthèse 10.

Dans le cas d'une ostéotomie de valgisation tibiale d'ouverture interne, le dispositif chirurgical de guidage 1 vient prendre place au niveau de la zone antéro-interne du tibia.

Dans ce cas, tel que représenté sur la figure 7, la patte 45 de la première pièce 4 vient alors appuyer sur l'insertion du tendon rotulien T, au niveau de la tubérosité tibiale antérieure ; les pattes 57 de la seconde pièce 5 permettent un bon plaquage du dispositif chirurgical de guidage 1 sur l'os et un rattrapage du jeu, afin que la patte 45 précitée vienne se plaquer efficacement sur la face postérieure du tendon rotulien T (réglage de position perpendiculaire à l'axe S' de l'os S).

La protrusion supérieure 48 (située en haut du dispositif chirurgical de guidage 1) permet un premier ajustement visuel en hauteur du dispositif chirurgical de guidage 1 (vérification de l'altitude du guide 1).

Ensuite, la broche supérieure 81 (voir la broche additionnelle 83) permet de contrôler et de valider ce bon positionnement du dispositif chirurgical de guidage 1 via une visualisation avec un amplificateur de brillance

La broche supérieure 81, insérée dans la seconde pièce 5 du dispositif chirurgical de guidage 1 et dans l'os S au travers du conduit tubulaire supérieur 581, fige provisoirement le dispositif chirurgical de guidage 1 sur l'os.

De manière optionnelle, la broche additionnelle 83, insérée dans la première pièce 4 du dispositif chirurgical de guidage 1 et dans l'os S au travers du conduit tubulaire 49 dédié, participe également à figer provisoirement le dispositif chirurgical de guidage 1 sur l'os.

Cette broche supérieure 81 (voire aussi la broche additionnelle 83) permet également une vérification de l'altitude du dispositif chirurgical de guidage 1, en accord avec le dossier de planification. Pour cela, le trajet de cette broche supérieure 81 (voire aussi la broche additionnelle 83), radio-opaque, est superposée avec le trajet théorique déterminé lors de la simulation, cette information figurant dans le dossier de planification opératoire fourni au chirurgien.

L'étape de préparation comprend encore une étape de « méchage », c'est-à-dire une étape de formation de trous dans l'os S en regard de chacun des orifices traversants 42, 56 du dispositif chirurgical de guidage 1.

Par exemple, les trous sont, respectivement, borgnes au niveau des orifices traversants 42 (proximaux) de la première pièce 4 et, d'autre part, traversants (bicorticaux) au niveau des orifices traversants 56 (distaux) de la seconde pièce 5.

Ce dispositif chirurgical de guidage 1 est ensuite fixé temporairement contre la surface de l'os S, au moyen de pins 7 rapportés au travers de certains au moins des orifices traversants 42, 56 (figure 8).

Cette fixation temporaire permet un maintien robuste du dispositif chirurgical de guidage 1, dans le but d'assurer une coupe très précise au sein de la fenêtre de guidage 2.

Lorsque cette étape de préparation est terminée, une étape d'ostéotomie de l'os peut être effectuée.

Le début de la coupe se fait grâce à la fenêtre de guidage 2, agencée sur mesure et dédiée à l'épaisseur connue au préalable de la lame de coupe 9 (figure 9).

La broche supérieure 81 (voire aussi la broche additionnelle 83) permet en plus de suivre le trajet de la lame de coupe 9. Cette broche supérieure 81 (voire aussi la broche additionnelle 83) permet de vérifier visuellement qu'il n'y a pas de perte de la bonne direction de la coupe (par exemple si ajustement trop large entre l'épaisseur de la lame et celle de la fenêtre).

Une broche inférieure 82 (dite encore « première broche inférieure 82 ») est ensuite insérée dans l'os, guidée par le conduit tubulaire inférieur 582 (figure 10).

Cette broche inférieure 82 est agencée pour intersecter la charnière du foyer d'ostéotomie (le foyer représente l'espace vide qui va être créé lors de l'ouverture du tibia rendue possible par le trait de scie). En d'autres termes, cette broche inférieure 82 coupe un axe de rotation A (figure 15), s'étendant de la partie postérieure jusqu'à la partie antérieure du tibia, qui représente la ligne de fuite de la rotation autour de laquelle est pratiqué l'ouverture ; cette broche inférieure 82 s'étend en outre avantageusement perpendiculairement, ou au moins approximativement perpendiculairement, par rapport à cet axe de rotation A.

Cet agencement assure encore une protection de la charnière lors du mouvement d'ouverture, servant de « guide » de déformation et évitant toute rupture.

Elle forme encore une butée de coupe mais également l'indication de la position idéale de la charnière.

Selon le second mode de réalisation, une seconde broche inférieure 84 peut être insérée dans l'os, guidée par le troisième conduit tubulaire 583 (figures 14 à 16).

Cette seconde broche inférieure 84 est également agencée pour intersecter la charnière du foyer d'ostéotomie, à proximité du point d'intersection de la première broche inférieure 82. En d'autres termes, là encore, cette seconde broche inférieure 84 coupe l'axe de rotation A (figure 15) qui représente la ligne de fuite de la rotation autour de laquelle est pratiqué l'ouverture ; cette seconde broche inférieure 84 s'étend en outre avantageusement perpendiculairement, ou au moins approximativement perpendiculairement, par rapport à cet axe de rotation A.

A ce sujet, cette seconde broche inférieure 84 est avantageusement strictement parallèle à la première broche inférieure 82. Cet agencement permet à la seconde broche inférieure 84 de franchir l'axe de rotation de la charnière exactement dans les mêmes conditions que la première broche inférieure 82.

De préférence, le point d'intersection de la seconde broche inférieure 84 au sein de la charnière se situe dans une portion centrale L1 de la longueur L de cette charnière (figure 15).

Un tel agencement vise à équilibrer, dans la profondeur du plan frontal, l'emplacement de la seconde broche inférieure 84 pour que la vis coulisse sur cette dernière et rééquilibre les forces de fixation en cas d'une rupture de charnière.

Par exemple, cette portion centrale L1 (centrée sur la longueur de la charnière L) a une longueur correspondant à 1/3 (voire 1/4, éventuellement 1/5) de la longueur de ladite charnière.

Dans une variante de réalisation, la première broche inférieure 82 peut être centrée sur la longueur de la charnière. Dans ce cas, la seconde broche inférieure 84 est déportée sur la longueur de cette charnière, tout en restant au plus près de cette première broche inférieure 82.

En pratique, la seconde broche inférieure 84 est insérée après la finition de la coupe. Elle peut également être utile pour récupérer l'intégrité de la charnière si celle-ci venait à céder malgré la présence de la première broche inférieure 82.

De plus, sa présence et sa visualisation au-delà de la coupe et au-delà de la corticale latérale permet, si cela est nécessaire, de venir par la voie externe, d'enfiler la vis canulée et de renforcer / synthétiser la charnière externe.

La première pièce 4 peut ensuite être retirée après extraction des pins 7 de maintien (figure 10).

La lame de coupe 9 va pouvoir s'appuyer sur la pièce distale 5 toujours fixée sur l'os, et en particulier sur sa patte supérieure 571, afin de continuer à suivre le trajet idéal.

L'opérateur peut alors aller chercher le point de charnière en venant buter mécaniquement sur la première broche inférieure 82 qui s'étend au niveau du foyer d'ostéotomie.

La charnière entre les deux fragments osseux Sp, Sd est ainsi protégée par cette première broche inférieure 82 (voire aussi par la seconde broche inférieure 84) qui traverse le trait de coupe.

La première broche inférieure 82 (voire aussi la seconde broche inférieure 84) est laissée jusqu'à la fin de l'intervention lorsque la plaque sera totalement solidaire de l'os. Ainsi toutes les manipulations intermédiaires traumatisant la charnière, et qui risqueraient de la fragiliser (voire de la rompre), sont préservées grâce la présence constante de cette première broche inférieure 82 (voire aussi de la seconde broche inférieure 84).

A noter que, une fois que la seconde pièce 5 (distale) est retirée, elle peut coulisser le long de cette première broche inférieure 82 (voire aussi de la seconde broche inférieure 84) de sorte à ce qu'elle(s) reste(ent) en place. La première broche inférieure 82 (voire aussi la seconde broche inférieure 84) joue ainsi un rôle « d'absorbeur de contrainte » et protège l'os de risque de fracture, pouvant créer la séparation des deux fragments osseux.

Une fois le trait de coupe terminé, la seconde pièce 5 est également retirée, tout en conservant en position inchangée la broche inférieure 82 (voire aussi la seconde broche inférieure 84).

La plaque d'ostéosynthèse 10 est positionnée en réinsérant les pins 7 au travers de ses orifices traversants distaux 1012 et de sorte qu'ils pénètrent dans les trous du fragment distal Sd obtenus grâce à la seconde pièce 5 (figure 11).

Le mouvement d'ouverture osseuse est réalisé par l'opérateur. Ce mouvement d'ouverture est par exemple réalisé au moyen d'un coin inséré entre les deux fragments osseux Se et Sd (ou de tout autre instrument adéquat, tel qu'une pince de Méhari).

La correction est atteinte dans tous les plans lorsque les trous du fragment proximal osseux Sp (obtenus par la première pièce 4) se retrouvent en face des orifices traversants proximaux 1011 de la plaque d'ostéosynthèse 10 (figure 11).

En effet, comme précisé ci-dessus, l'agencement relatif entre les deux groupes d'orifices traversants 42, 56 du dispositif chirurgical de guidage 1 tient compte, d'une part, de la correction destinée à être appliquée lors de la technique d'ostéotomie d'ouverture et, d'autre part, de l'agencement relatif des deux groupes d'orifices traversants 1011, 1012 de la plaque d'ostéosynthèse 10.

Des pins 7 sont alors insérés au travers de ces orifices traversants proximaux 1011 pour maintenir l'ouverture (figure 12).

Les différentes vis de fixation 102 sont ensuite insérées successivement dans l'os S, au travers des différents orifices 101 de la plaque d'ostéosynthèse 10.

Lorsque la plaque d'ostéosynthèse 10 est définitivement fixée, s'il le souhaite, l'opérateur peut combler l'ouverture obtenue avec par exemple des substituts osseux synthétiques solides ou injectables, des greffes osseuses (greffons iliaques).

S'il le souhaite, après ouverture, l'opérateur peut aussi utiliser la seconde broche inférieure 84 pour guider l'insertion d'une vis canulée 103 au travers de la charnière (figure 16).

Cette vis canulé 103 participe ainsi au maintien de l'ouverture (voire aussi du substitut osseux), en association avec la plaque d'ostéosynthèse 10.

Cette vis canulé 103 a également pour vocation de renforcer l'intégrité de la charnière, et ainsi de réduire encore le risque de rupture post-opératoire. Elle permet également de participer à l'intégrité de la charnière et donc à la bonne valeur d'ouverture définie par le dispositif chirurgical de guidage 1.

Une telle vis canulé 103 peut également être intéressante dans le cadre d'une réparation peropératoire, afin de réparer une charnière qui aurait cédée malgré la première broche inférieure 82 (suite à geste trop brusque, ouverture de grande valeur, etc.).

De manière générale, le dispositif chirurgical de guidage 1 aide ainsi le chirurgien à effectuer son intervention de manière précise et sécuritaire.

Ce dispositif chirurgical de guidage a l'intérêt d'assister l'opérateur tout au long de l'intervention d'ostéotomie d'ouverture (notamment pour la réalisation du trait de coupe et de l'ouverture), de manière à suivre précisément la planification préopératoire.

Ce dispositif chirurgical de guidage peut être utilisé pour mettre en oeuvre une technique d'ostéotomie d'ouverture sur d'autres os, par exemple le fémur distal, le poignet, le tibia distal.

Ce dispositif donne encore un accès à des corrections complexes dans les trois dimensions, avec un contrôle continu du processus opératoire.

## Revendications

1. Dispositif chirurgical de guidage, destiné à être rapporté contre un os d'un patient, pour assister une technique d'ostéotomie d'ouverture,
lequel dispositif chirurgical de guidage (1) est **caractérisé en ce qu'**il comprend :
- une face arrière (11) adaptée à être positionnée contre une surface dudit os (S), et
- une fenêtre (2) pour le guidage d'un trait de coupe destiné à être réalisé dans ledit os (S),
laquelle fenêtre de guidage (2) est délimitée par un bord supérieur (21) et par un bord inférieur (22), ménagés en regard et à distance l'un de l'autre,
lequel dispositif chirurgical de guidage (1) comprend deux pièces (4, 5) qui sont assemblées l'une avec l'autre par le biais de moyens solidarisation amovible, à savoir :
- une pièce proximale (4), comportant une bordure inférieure (413) définissant au moins une partie dudit bord supérieur (21) de ladite fenêtre de guidage (2), et
- une pièce distale (5), comportant une bordure supérieure (54) définissant au moins une partie dudit bord inférieur (22) de ladite fenêtre de guidage (2),
lesquelles pièces proximale (4) et distale (5) sont chacune munies de plusieurs orifices traversants (42, 56) qui débouchent au travers de la face arrière (11), adaptés à accueillir des organes (7) implantables temporairement dans ledit os (S) et/ou adaptés à guider un perçage de trous dans ledit os (S),
la pièce distale (5) comporte des conduits tubulaires (58) qui sont adaptés à guider l'insertion d'une broche au sein de l'os (S),
**caractérisé en ce que** les conduits tubulaires (58) comprennent au moins deux conduits tubulaires distaux (582, 583) qui sont ménagés à distance de la fenêtre de guidage (2) et qui sont orientés de sorte que leurs axes longitudinaux (582', 583') respectifs coupent le plan (2') de la fenêtre de guidage (2) pour positionner chacun une broche (82, 84) de butée de coupe et/ou d'indication de charnière.

2. Dispositif chirurgical de guidage, selon la revendication 1, **caractérisé en ce qu'**il comporte deux bordures latérales (13) dont l'une au moins comporte au moins une patte (45, 57) adaptée à être positionnée contre une surface dudit os (S), laquelle patte (45, 57) présente une capacité de déformation élastique.

3. Dispositif chirurgical de guidage, selon l'une quelconque des revendications 1 ou 2, **caractérisé en ce que** :
- la pièce proximale (4) comporte au moins deux orifices traversants (42) juxtaposés, indépendants, qui s'étendent dans une bande virtuelle (42') destinée à être orientée transversalement par rapport à l'axe longitudinal (S') de l'os (S), et
- la pièce distale (5) comporte au moins deux orifices traversants (56) juxtaposés, indépendants, qui s'étendent dans une bande virtuelle (56') destinée à être orientée au moins approximativement parallèlement à l'axe longitudinal (S') de l'os (S).

4. Dispositif chirurgical de guidage, selon l'une quelconque des revendications 1 à 3, **caractérisé en ce que** les conduits tubulaires (58) comprennent un conduit tubulaire proximal (581) qui est ménagé directement sous-jacent de la fenêtre de guidage (2) et qui est orienté de sorte que son axe longitudinal (581') soit parallèle au plan (2') de la fenêtre de guidage (2) pour positionner une broche (81) participant au guidage du trait de coupe.

5. Dispositif chirurgical de guidage, selon l'une quelconque des revendications 1 à 4, **caractérisé en ce que** la pièce proximale (4) est dotée d'une protrusion supérieure (48), avantageusement destinée à aider le praticien à positionner correctement le dispositif chirurgical de guidage (1) à la bonne distance par rapport au plateau tibial d'un patient.

6. Dispositif chirurgical de guidage, selon l'une quelconque des revendications 1 à 5, **caractérisé en ce qu'**il est réalisé dans un matériau plastique adapté à la fabrication additive.

7. Matériel pour la mise en oeuvre d'une technique d'ostéotomie d'ouverture, **caractérisé en ce qu'**il comprend :
- un dispositif chirurgical de guidage (1), selon l'une quelconque des revendications 1 à 6,
- des organes (7) implantables temporairement dans ledit os (S), au travers des orifices traversants (42, 56) dudit dispositif chirurgical de guidage (1),
- des broches (8) implantables temporairement dans ledit os (S), au travers des conduits traversants (58) dudit dispositif chirurgical de guidage (1),
- une lame de coupe (9), pour réaliser le trait de coupe guidé par le dispositif chirurgical de guidage (1),
- une plaque d'ostéosynthèse (10) munie de deux groupes d'orifices traversants (1011, 1012), l'un proximal et l'autre distal, chacun desdits groupes (1011, 1012) étant destinés à accueillir des vis de fixation (102) destinées à être implantées dans l'un des deux fragments osseux,
- un jeu de vis (102) pour la fixation dans l'os (S), destinées à être insérées au travers des orifices traversants (1011, 1012) de ladite plaque d'ostéosynthèse (10) pour fixer cette dernière à la surface de l'os (S),
**en ce que** l'agencement des orifices traversants (42) de la pièce proximale (4) correspond à l'agencement des orifices traversants proximaux (1011) de la plaque d'ostéosynthèse (10),
**en ce que** l'agencement des orifices traversants (56) de la pièce distale (5) correspond à l'agencement des orifices traversants distaux (1012) de la plaque d'ostéosynthèse (10), et
**en ce que** l'agencement relatif entre les deux groupes d'orifices traversants (42, 56) du dispositif chirurgical de guidage (1) est fonction, d'une part, de la correction destinée à être appliquée lors de la technique d'ostéotomie d'ouverture et, d'autre part, de l'agencement relatif des deux groupes d'orifices traversants (1011, 1012) de la plaque d'ostéosynthèse (10).

8. Procédé pour l'obtention d'un dispositif chirurgical de guidage selon l'une quelconque des revendications 1 à 6, **caractérisé en ce qu'**il comprend :
a) une étape de simulation de la correction destinée à être appliquée à un os (S) par une technique d'ostéotomie d'ouverture à partir des données suivantes :
- de données anatomiques relevées sur l'os (S) à traiter,
- de données relatives à la plaque d'ostéosynthèse (10) à poser, et
- de données de planification préopératoire, relatives à la correction destinée à être appliquée à cet os (S) par la technique d'ostéotomie d'ouverture,
b) une étape de fabrication dudit dispositif chirurgical de guidage (1), tenant compte des données de correction issues de ladite étape de simulation.

9. Procédé selon la revendication 8, **caractérisé en ce que** l'étape de fabrication dudit dispositif chirurgical de guidage (1) consiste en une étape de fabrication additive.

## Patentansprüche

1. Chirurgische Führungsvorrichtung, die dazu bestimmt ist, an einem Knochen eines Patienten angebracht zu werden, um eine Open-Wedge-Osteotomie zu unterstützen, wobei die chirurgische Führungsvorrichtung (1) **dadurch gekennzeichnet ist, daß** sie
- eine Rückseite (11), die dazu ausgelegt ist, auf einer Oberfläche des Knochens (S) angeordnet zu werden, und
- ein Fenster (2) zum Führen einer Schnittlinie, die im Knochen (S) ausgeführt werden soll,
aufweist,
wobei das Führungsfenster (2) durch einen oberen Rand (21) und durch einen unteren Rand (22) begrenzt ist, die einander gegenüberliegend und in einem Abstand voneinander eingerichtet sind,
wobei die chirurgische Führungsvorrichtung (1) zwei Stücke (4, 5) aufweist, die mittels zweier abnehmbarer Befestigungsmittel miteinander verbunden sind, und zwar:
- ein proximales Stück (4), das einen unteren Rand (413) aufweist, der mindestens einen Teil des oberen Rands (21) des Führungsfensters (2) definiert, und
- ein distales Stück (5), das einen oberen Rand (54) aufweist, der mindestens einen Teil des unteren Rands (22) des Führungsfensters (2) definiert,
wobei das proximale (4) und das distale (5) Stück jeweils mit mehreren Durchgangslöchern (42, 56) versehen sind, die durch die Rückseite (11) hindurch münden, dazu ausgelegt sind, die in den Knochen (S) einzusetzenden einsetzbaren Organe (7) vorübergehend aufzunehmen und/oder dazu ausgelegt sind, ein Bohren von Löchern im Knochen (S) zu führen,
- wobei das distale Stück (5) rohrförmige Leitungen (58) aufweist, die dazu ausgelegt sind, das Einführen einer Nadel in den Knochen (S) zu führen,
**dadurch gekennzeichnet, daß** die rohrförmigen Leitungen (58) mindestens zwei distale Rohrleitungen (582, 583) aufweisen, die in einem Abstand vom Führungsfenster (2) angeordnet sind und die so ausgerichtet sind, daß deren jeweilige Längsachsen (582', 583') die Ebene (2') des Führungsfensters (2) schneiden, um jeweils einen Schnittanschlagsstift und/oder Scharnieranzeigestift (82, 84) zu positionieren.

2. Chirurgische Führungsvorrichtung gemäß Anspruch 1, **dadurch gekennzeichnet, daß** sie zwei Seitenränder (13) aufweist, von denen mindestens einer einen Bügel (45, 57) aufweist, der dazu ausgelegt ist, an einer Oberfläche des Knochens (S) angesetzt zu werden, wobei der Bügel (45, 57) elastisch verformbar ist.

3. Chirurgische Führungsvorrichtung gemäß einem der Ansprüche 1 oder 2, **dadurch gekennzeichnet, daß** .
- das proximale Stück (4) mindestens zwei nebeneinander liegende, voneinander unabhängige Durchgangslöcher (42) aufweist, die sich in einem virtuellen Band (42') erstrecken, das quer zur Längsachse (S') des Knochens (S) ausgerichtet sein soll, und
- das distale Stück (5) mindestens zwei nebeneinander liegende, voneinander unabhängige Durchgangslöcher (42) aufweist, die sich in einem virtuellen Band (56') erstrecken, das mindestens ungefähr parallel zur Längsachse (S') des Knochens (S) ausgerichtet sein soll.

4. Chirurgische Führungsvorrichtung gemäß einem der Ansprüche 1 bis 3, **dadurch gekennzeichnet, daß** die rohrförmigen Leitungen (58) eine proximale rohrförmige Leitung (581) aufweisen, die direkt unter dem Führungsfenster (2) ausgebildet ist und die so ausgerichtet ist, daß deren Längsachse (581 `) parallel zur Ebene (2') des Führungsfensters (2) ist, um einen an der Führung der Schnittlinie beteiligten Stift (81) zu positionieren.

5. Chirurgische Führungsvorrichtung gemäß einem der Ansprüche 1 bis 4, **dadurch gekennzeichnet, daß** das proximale Stück (5) mit einer oberen Ausstülpung (48) versehen ist, die vorteilhafterweise dazu bestimmt ist, dem Arzt zu helfen, die chirurgische Führungsvorrichtung (1) im richtigen Abstand zum Tibiaplateau eines Patienten zu positionieren.

6. Chirurgische Führungsvorrichtung gemäß einem der Ansprüche 1 bis 5, **dadurch gekennzeichnet, daß** sie aus einem plastischen Material hergestellt ist, das für eine additive Herstellung geeignet ist.

7. Material für die Durchführung einer Open-Wedge-Osteotomie, **dadurch gekennzeichnet,**
**daß** es
- eine chirurgische Führungsvorrichtung gemäß einem der Ansprüche 1 bis 6,
- Organe (7), die durch Durchgangslöcher (42, 56) der chirurgischen Führungsvorrichtung (1) hindurch vorübergehend in den Knochen (S) einsetzbar sind,
- Stifte (8), die durch durchgehende Leitungen (58) der chirurgischen Führungsvorrichtung (1) hindurch vorübergehend in den Knochen (S) einsetzbar sind,
- eine Klinge (9) zur Ausführung der durch die chirurgische Führungsvorrichtung (1) geführten Schnittlinie,
- eine Osteosyntheseplatte (10), die mit zwei Gruppen Durchgangslöchern (1011, 1012) versehen ist, einer proximalen und einer distalen Gruppe, wobei jede dieser Gruppen (1011, 1012) dazu bestimmt ist, Befestigungsschrauben (102) aufzunehmen, die dazu bestimmt sind, in eins der beiden Knochenfragmente eingesetzt zu werden,
- einen Satz Schrauben (102) für die Befestigung im Knochen (S), die dazu bestimmt sind, durch die Durchgangslöcher (1011, 1012) der Osteosyntheseplatte (10) hindurch eingesetzt zu werden, um letztere an der Oberfläche des Knochens (S) zu befestigen,
aufweist,
**daß** die Anordnung der Durchgangslöcher (42) des proximalen Stücks (4) der Anordnung der proximalen Durchgangslöcher (1011) der Osteosyntheseplatte (10) entspricht,
**daß** die Anordnung der Durchgangslöcher (56) des distalen Stücks (5) der Anordnung der distalen Durchgangslöcher (1012) der Osteosyntheseplatte (10) entspricht, und
**daß** die relative Anordnung der beiden Gruppen Durchgangslöcher (42, 56) der chirurgischen Führungsvorrichtung (1) zueinander einerseits von der Korrektur, die bei der Open-Wedge-Osteotomie vorgenommen werden soll, und andererseits von der relativen Anordnung der beiden Gruppen Durchgangslöcher (1011, 1012) der Osteosyntheseplatte (10) zueinander abhängt.

8. Verfahren zum Erhalten einer chirurgischen Führungsvorrichtung gemäß einem der Ansprüche 1 bis 6, **dadurch gekennzeichnet, daß** es
a) einen Schritt der Simulation der Korrektur, die an einem Knochen (S) durch eine Open-Wedge-Osteotomie vorgenommen werden soll, auf der Grundlage der folgenden Angaben:
- an dem zu behandelnden Knochen (S) abgenommene anatomische Daten,
- Daten bezüglich der zu setzenden Osteosyntheseplatte (10) und
- Vorbereitungsplanungsdaten bezüglich der durch die Open-Wedge-Osteotomie an dem Knochen (S) vorzunehmenden Korrektur,
b) einen Schritt der Herstellung der chirurgischen Führungsvorrichtung (1) unter Berücksichtigung der aus dem Simulationsschritt hervorgegangenen Daten aufweist.

9. Verfahren gemäß Anspruch 8, **dadurch gekennzeichnet, daß** der Schritt der Herstellung der chirurgischen Führungsvorrichtung (1) aus einem Schritt der additiven Herstellung besteht.

## Claims

1. A surgical guide device, intended to be placed against a patient's bone, for assisting an opening wedge osteotomy technique,
said surgical guide device (1) being **characterized in that** it comprises:
- a rear face (11) adapted to be positioned against a surface of said bone (S), and
- a window (2) for guiding a cut line intended to be made into said bone (S),
wherein said guide window (2) is delimited by an upper edge (21) and a lower edge (22), arranged opposite to each other and spaced apart from each other,
wherein said surgical guide device (1) comprises two parts (4, 5) that are assembled to each other through detachable fastening means, i.e.:
- a proximal part (4), having a lower edge (413) defining at least one portion of said upper edge (21) of said guide window (2), and
- a distal part (5), having an upper edge (54) defining at least one portion of said lower edge (22) of said guide window (2),
wherein said proximal (4) and distal (5) parts are each provided with several through-orifices (42, 56) opening through the rear face (11), adapted to receive elements (7) temporarily implantable into said bone (S) and/or adapted to guide a drilling of holes into said bone (S),
the distal part (5) comprises tubulars ducts (58) that are adapted to guide the insertion of a rod within the bone (S),
**characterized in that** the distal part (5) comprises at least two distal tubular ducts (582, 583) that are arranged remote from the guide window (2) and that are directed in such a manner that their respective longitudinal axes (582', 583') intersect the plane (2') of the guide window (2) in order to each position a cut stop and/or hinge indication rod (82, 84).

2. The surgical guide device according to claim 1, **characterized in that** it has two lateral edges (13), at least one of which comprises at least one tab (45, 57) adapted to be positioned against a surface of said bone (S), wherein said tab (45, 57) has an ability of elastic deformation.

3. The surgical guide device according to any one of claims 1 or 2, **characterized in that**:
- the proximal part (4) comprises at least two through-orifices (42), juxtaposed and independent of each other, that extend in a virtual strip (42') intended to be directed transversally with respect to the longitudinal axis (S') of the bone (S), and
- the distal part (5) comprises at least two through-orifices (56), juxtaposed and independent of each other, extending in a virtual strip (56') intended to be directed at least approximately parallel to the longitudinal axis (S') of the bone (S).

4. The surgical guide device according to any one of claims 1 to 3, **characterized in that** the tubular ducts (58) comprise a proximal tubular duct (581) that is arranged directly underlying the guide window (2) and that is directed in such a manner that its longitudinal axis (581') is parallel to the plane (2') of the guide window (2) in order to position a rod (81) helping in the guiding of the cut line.

5. The surgical guide device according to any one of claims 1 to 4, **characterized in that** the proximal part (4) is provided with an upper protrusion (48), advantageously intended to help the practitioner to correctly position the surgical guide device (1) at the good distance with respect to the tibial plateau of a patient.

6. The surgical guide device according to any one of claims 1 to 5, **characterized in that** it is made of a plastic material adapted to the additive manufacturing.

7. Equipment for implementing an opening wedge osteotomy technique, **characterized in that** it comprises:
- a surgical guide device (1) according to any one of claims 1 to 6,
- elements (7) temporarily implantable into said bone (S), through the through-orifices (42, 56) of said surgical guide device (1),
- rods (8) temporarily implantable into said bone (S), through the through-ducts (58) of said surgical guide device (1),
- a cutting blade (9), for making the cut line guided by the surgical guide device (1),
- an osteosynthesis plate (10) provided with two groups of through-orifices (1011, 1012), a proximal one and a distal one, each of said groups (1011, 1012) being intended to receive fastening screws (102) intended to be implanted into one of the two bone fragments,
- a set of screws (102) for the fastening to the bone (S), intended to be inserted through the through-orifices (1011, 1012) of said osteosynthesis plate (10) to fasten the latter to the surface of the bone (S),
**in that** the arrangement of the through-orifices (42) of the proximal part (4) corresponds to the arrangement of the proximal through-orifices (1011) of the osteosynthesis plate (10),
**in that** the arrangement of the through-orifices (56) of the distal part (5) corresponds to the arrangement of the distal through-orifices (1012) of the osteosynthesis plate (10), and
**in that** the relative arrangement between the two groups of through-orifices (42, 56) of the surgical guide device (1) is function, on the one hand, of the correction intended to be applied during the opening wedge osteotomy technique and, on the other hand, of the relative arrangement of the two groups of through-orifices (1011, 1012) of the osteosynthesis plate (10).

8. A method for obtaining a surgical guide device according to any one of claims 1 to 6, **characterized in that** it comprises:
a) a step of simulating the correction intended to be applied to a bone (S) by an opening wedge osteotomy technique from the above-mentioned data:
- anatomic data taken on the bone (S) to be treated,
- data relating to the osteosynthesis plate (10) to be installed, and
- preoperative planning data, relating to the correction intended to be applied to this bone (S) by the opening wedge osteotomy technique,
b) a step of manufacturing said surgical guide device (1), taking into account the correction data coming from said simulation step.

9. The method according to claim 8, **characterized in that** the step of manufacturing said surgical guide device (1) consists of a step of additive manufacturing.
